# EUROPÄISCHE PATENTSCHRIFT

(11) **EP 1 929 944 B1**
(45) Veröffentlichungstag und Bekanntmachung des Hinweises auf die Patenterteilung: **29.12.2010**
(21) Anmeldenummer: 07121388.8
(22) Anmeldetag: 23.11.2007
(51) Int. Cl.: A61B 5/107, G01B 9/021, G03H 1/04, H04R 25/00, G01B 11/24

(54) **Ohrkanalhologramm für Hörvorrichtungen**
Ear canal hologram for hearing devices
Hologramme de canal auditif pour dispositifs auditifs

(30) Priorität: 04.12.2006 DE 102006057099
(43) Veröffentlichungstag der Anmeldung: 11.06.2008
(73) Patentinhaber: Siemens Audiologische Technik GmbH, 91058 Erlangen (DE)
(72) Erfinder: Reithinger, Jürgen, 91077 Neunkirchen am Brand (DE)
(74) Vertreter: Maier, Daniel Oliver

(56) Entgegenhaltungen:
- WO-A-03/105685
- US-A- 4 611 288
- US-A1- 2006 276 709
- US-B1- 7 127 109

## Beschreibung

Die vorliegende Erfindung betrifft eine Aufnahmevorrichtung zur Aufnahme der räumlichen Gestalt zumindest eines Teils eines Ohrkanals oder Ohrabdrucks. Darüber hinaus betrifft die vorliegende Erfindung eine Einrichtung zur Herstellung einer Gehäuseschale oder einer Otoplastik für eine Hörvorrichtung sowie ein entsprechendes Aufnahme- und ein Herstellungsverfahren. Unter einer Hörvorrichtung wird hier insbesondere ein Hörgerät, aber auch ein Headset, Kopfhörer und dergleichen verstanden.

Hörgeräte sind tragbare Hörvorrichtungen, die zur Versorgung von Schwerhörenden dienen. Um den zahlreichen individuellen Bedürfnissen entgegenzukommen, werden unterschiedliche Bauformen von Hörgeräten wie Hinter-dem-Ohr-Hörgeräte (HdO), Indem-Ohr-Hörgeräte (IdO) und Concha-Hörgeräte bereitgestellt. Die beispielhaft aufgeführten Hörgeräte werden am Außenohr oder im Gehörgang getragen. Darüber hinaus stehen auf dem Markt aber auch Knochenleitungshörhilfen, implantierbare oder vibrotaktile Hörhilfen zur Verfügung. Dabei erfolgt die Stimulation des geschädigten Gehörs entweder mechanisch oder elektrisch.

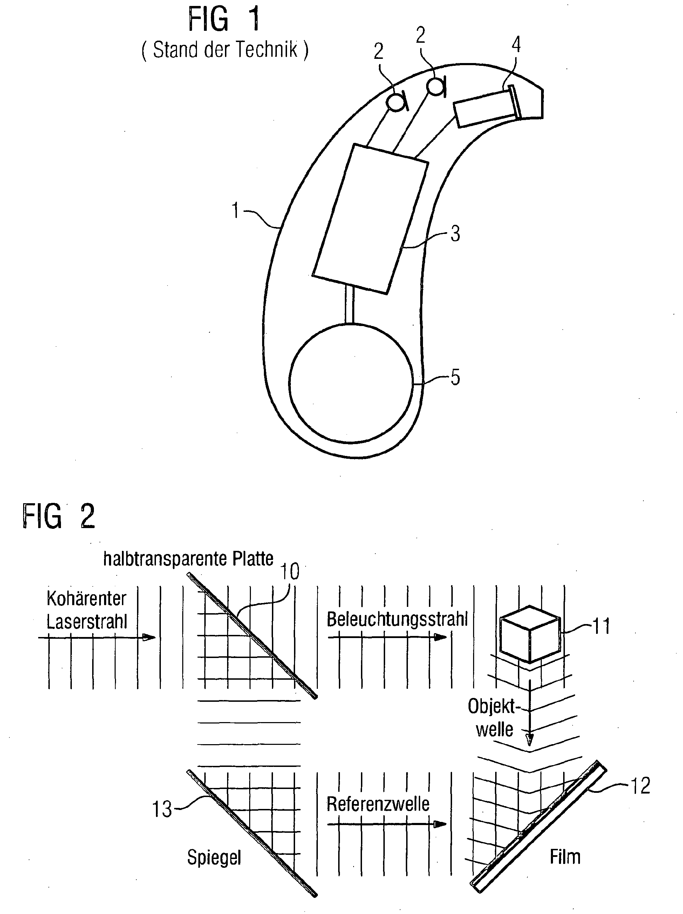

Hörgeräte besitzen prinzipiell als wesentliche Komponenten einen Eingangswandler, einen Verstärker und einen Ausgangswandler. Der Eingangswandler ist in der Regel ein Schallempfänger, z. B. ein Mikrofon, und/oder ein elektromagnetischer Empfänger, z. B. eine Induktionsspule. Der Ausgangswandler ist meist als elektroakustischer Wandler, z. B. Miniaturlautsprecher, oder als elektromechanischer Wandler, z. B. Knochenleitungshörer, realisiert. Der Verstärker ist üblicherweise in eine Signalverarbeitungseinheit integriert. Dieser prinzipielle Aufbau ist in FIG 1 am Beispiel eines Hinter-dem-Ohr-Hörgeräts dargestellt. In ein Hörgerätegehäuse 1 zum Tragen hinter dem Ohr sind ein oder mehrere Mikrofone 2 zur Aufnahme des Schalls aus der Umgebung eingebaut. Eine Signalverarbeitungseinheit 3, die ebenfalls in das Hörgerätegehäuse 1 integriert ist, verarbeitet die Mikrofonsignale und verstärkt sie. Das Ausgangssignal der Signalverarbeitungseinheit 3 wird an einen Lautsprecher bzw. Hörer 4 übertragen, der ein akustisches Signal ausgibt. Der Schall wird gegebenenfalls über einen Schallschlauch, der mit einer Otoplastik im Gehörgang fixiert ist, zum Trommelfell des Geräteträgers übertragen. Die Stromversorgung des Hörgeräts und insbesondere die der Signalverarbeitungseinheit 3 erfolgt durch eine ebenfalls ins Hörgerätegehäuse 1 integrierte Batterie 5.

Zur Anfertigung von IdO-Hörgeräten werden Verfahren benötigt, mit denen die Form des Gehörgangs möglichst genau auf die Gehäuseschale eines Ido-Hörgeräts übertragen werden kann. Üblicherweise wird ein Ohrabdruck eines Gehörgangs abgenommen, um daraus die Form für eine Gehäuseschale herzustellen. Seit einiger Zeit werden derartige Ohrabdrucke mittels Scannen in PC-Systeme eingelesen, um dann digital weiter verarbeitet zu werden. Diese Scanner benutzen meist das Triangulationsverfahren zum Messen der 3D-Daten der Objekte. Dazu wird eine Lichtquelle (Projektor) benutzt, die ein Muster projiziert. Dieses Muster wird von einer Kamera wieder aufgenommen, die in einem Winkel zum Projektor steht. Daraus kann die räumliche Tiefenstruktur ausgerechnet werden.

Weiterhin sind Verfahren bekannt, bei denen der Gehörgang direkt gescannt wird, ohne einen Ohrabdruck zu benötigen. Diese Scanner wie auch die zum Aufnehmen eines Ohrabdrucks sind relativ aufwändig und basieren fast ausschließlich auf Triangulationsmessmethoden, die präzise Optiken nötig machen und auch einen aufwändigen Abgleich des Systems erfordern.

Aus der Druckschrift WO 02/071794 A1 ist ein solches Verfahren zur Modellierung individueller Ohrstücke bekannt. Dabei wird ein 3D-Scanner eingesetzt, um ein virtuelles Modell des Ohrkanals zu gewinnen. Anhand des 3D-Modells wird dann das Ohrstück gefertigt.

Zur Herstellung von Gehäuseschalen oder Otoplastiken für Hörvorrichtungen eignet sich eine so genannte Rapid Prototyping & Herstellung. Unterschiedliche Verfahren hierzu sind beispielsweise in der Druckschrift DE 696 34 921 P2 beschrieben. Insbesondere werden in diesem Rahmen stereolitographische Verfahren dazu eingesetzt, um Kunststoffhörgeräteschalen oder Otoplastiken zu fertigen.

Aus der Druckschrift WO 03/105685 A2 ist ein Gerät zur Bestimmung der Gestalt des Gehörgangs bekannt, das auf dem konoskopischen Prinzip basiert. Der von einem zu vermessenden Objekt reflektierte Strahl wird in einem konoskopischen Modul in einen ordentlichen und einen außerordentlichen Anteil aufgeteilt. Dadurch lässt sich ein Interferenzmuster gewinnen, aus dem der Abstand des Objektpunkts vom Sensor ermittelt werden kann. Bei dem vorgestellten Gerät wird ein beweglicher Spiegel, der den Beleuchtungs- und reflektierten Objektstrahl umlenkt, in den Ohrkanal eingeführt.

Weiterhin ist aus der Druckschrift EP 1 223 450 A2 ein Mikroskop bekannt. Zum Betrieb des Mikroskops ist ein Referenzobjekt für die Kalibrierung, Justierung und Einstellung des Mikroskops vorgesehen.

Die Druckschrift US 4,611,288 A beschreibt ein Gerät zur Aufnahme von Zähnen. Dabei wird mittels einer halbtransparenten Platte ein Hologramm des Zahnbereichs erstellt. Zwei bis drei optische Fasern, die das Licht führen werden hierzu in den Mund geführt. Die Aufnahmen werden zur automatischen Herstellung von Dentalprothesen verwendet.

Ferner offenbart die Druckschrift US 7,127,109 B1 ein digitales Interferenzholographiemikroskop. Es werden mehrere Hologramme mit unterschiedlichen Wellenlängen aufgenommen. Die Aufnahmen erfolgen durch digitale Kameras, so dass eine numerische Rekonstruktion möglich ist.

Die Aufgabe der vorliegenden Erfindung besteht darin, auf einfache und zuverlässige Weise Informationen über die räumliche Gestalt eines Ohrkanals zu gewinnen.

Erfindungsgemäß wird diese Aufgabe gelöst durch eine Aufnahmevorrichtung **nach Anspruch 1** oder ein Verfahren nach Anspruch 6.

Weiterhin ist erfindungsgemäß vorgesehen eine Einrichtung zur Herstellung einer Gehäuseschale oder einer Otoplastik für eine Hörvorrichtung mit einer oben dargestellten Aufnahmevorrichtung und einer Ausformungseinheit zum Formen der Gehäuseschale oder der Otoplastik aus dem durch die Aufnahmevorrichtung gewonnenen Hologramm des Ohrkanals.

Die oben genannte Aufnahmevorrichtung weist eine Führungseinrichtung auf, mit der die Holographieeinheit im Ohrkanal schwenkbar, rotierbar und längs des Ohrkanals verschiebbar ist. Damit kann der in sich gekrümmte Ohrkanal in gewünschter Weise aufgenommen werden.

Darüber hinaus ist es günstig, wenn durch die Holographieeinheit mehrere Einzelhologramme aufgenommen und zu dem Hologramm des Ohrkanals, d. h. einem Gesamthologramm, zusammengesetzt werden können. Damit kann der Ohrkanal bzw. der Ohrabdruck aus unterschiedlichen Perspektiven aufgenommen werden, so dass ein sehr genaues Hologramm des Ohrkanals erstellt werden kann.

Ferner kann die Holographieeinheit dafür geeignet sein, ein digitales Hologramm des Ohrkanals zu erstellen. Die digitalen Daten können dann unmittelbar für die Herstellung der Gehäuseschale bzw. der Otoplastik der Hörvorrichtung verwendet werden.

Die Holographieeinheit kann insbesondere einen CCD-Chip oder CMOS-Chip als Aufnahmesensor aufweisen. Hierdurch können bereits Aufnahmerohdaten in digitaler Form gewonnen werden.

Vorteilhaft ist darüber hinaus auch, wenn beim Aufnehmen des Ohrkanals für das Hologramm ein Referenzobjekt mit aufgenommen wird. Mit ihm lässt sich dann die Aufnahme kalibrieren, so dass sie für tatsächliche Messungen bzw. die Herstellung der Gehäuseschale oder der Otoplastik verwendet werden kann. Insbesondere kann anhand des Referenzobjekts eine automatische Kalibrierung durchgeführt werden. Damit können regelmäßig Kalibrierungen ohne Aufwand durchgeführt werden, so dass stets eine hohe Hologrammqualität gewährleistet werden kann.

Die vorliegende Erfindung ist anhand der beigefügten Zeichnungen näher erläutert, in denen zeigen:
- FIG 1: den prinzipiellen Aufbau eines Hörgeräts gemäß dem Stand der Technik;
- FIG 2: eine Prinzipdarstellung zur Gewinnung eines Holo- gramms und
- FIG 3: eine schematische Darstellung eines erfindungsgemä- ßen Hologrammaufnahmegeräts in einem Ohrkanal.

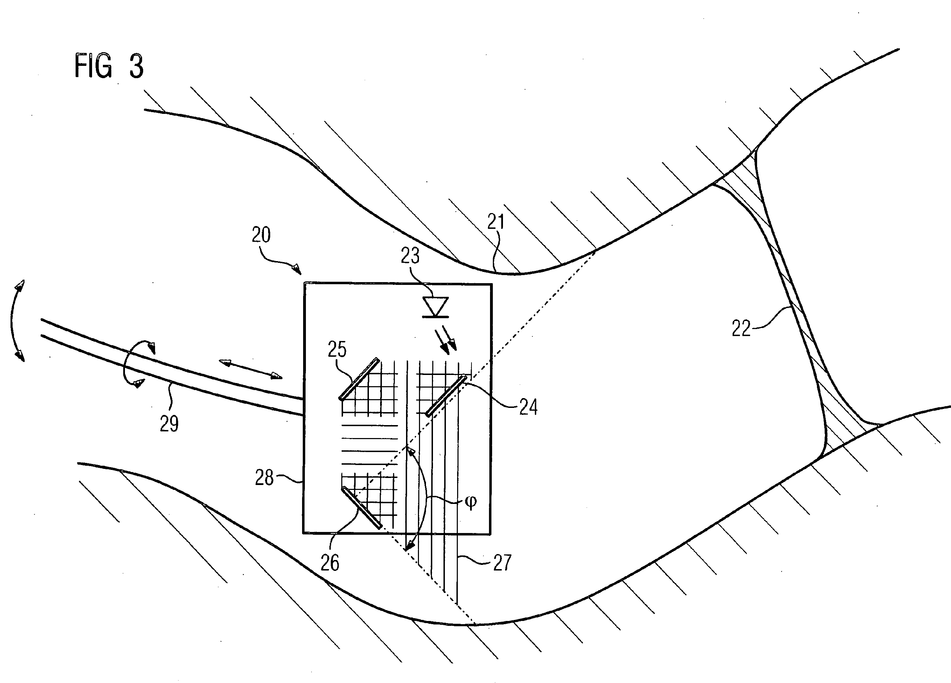

Das nachfolgend näher geschilderte Ausführungsbeispiel stellt eine bevorzugte Ausführungsform der vorliegenden Erfindung dar. Zunächst wird jedoch anhand von FIG 2 kurz das Prinzip der Holographie erläutert, um den Aufbau des erfindungsgemäßen Geräts gemäß FIG 3 besser zu verstehen.

Entsprechend FIG 2 wird zur Gewinnung eines Hologramms ein kohärenter Laserstrahl auf eine halbtransparente Platte 10 geschickt. Ein Teil des Laserstrahls durchdringt die halbtransparente Platte und bildet einen Beleuchtungsstrahl, der auf das Objekt 11, hier der Ohrkanal oder der Ohrabdruck, trifft. Ein Teil des Beleuchtungsstrahls wird an dem Objekt 11 reflektiert und bildet eine Objektwelle, die auf einen Film 12 auftrifft. Gleichzeitig hierzu wird von der halbtransparenten Platte 10 ein Teil des kohärenten Laserstrahls auf einen Spiegel 13 und von dort weiter auf den Film 12 als Referenzquelle umgelenkt. Auf dem Film 12 überlagert sich die Objektwelle mit der Referenzwelle, woraus das Hologramm resultiert. Um das Hologramm betrachten zu können, wird der belichtete und entwickelte Film wiederum mit einem kohärenten Laserstrahl bestrahlt.

Entsprechend der vorliegenden Erfindung wird nun diese Hologrammaufnahmetechnik dazu verwendet, die räumliche Gestalt eines Ohrkanals aufzunehmen bzw. zu vermessen. Hierzu wird das in FIG 3 schematisch dargestellte Hologrammaufnahmegerät 20 verwendet. Entsprechend der Darstellung von FIG 2 befindet sich dieses Aufnahmegerät 20 in einem Ohrkanal 21, der an seiner Innenseite durch ein Trommelfell 22 begrenzt ist. Der Ohrkanal 21 ist gekrümmt, so dass er von außen in seiner Gesamtheit nicht ohne weiteres einsichtig ist.

Das Aufnahmegerät 20 besitzt im Wesentlichen den Aufbau, der im Zusammenhang mit FIG 2 dargestellt wurde. Bei dem konkreten Aufbau von FIG 3 wird eine Laserdiode 23 als Lichtquelle für das kohärente Laserlicht verwendet. Ein Teil des Lichts wird über eine halbtransparente Platte 24 und einen Spiegel 25 zu einem Aufnahmesensor 26 umgelenkt. Ein anderer Teil des Laserlichts wird durch die halbtransparente Platte 24 hindurchgeführt und in den Ohrkanal 21 abgestrahlt. Der Übersicht halber sind die optischen Elemente zur Abstrahlung des Lichts in den Ohrkanal in FIG 3 nicht dargestellt. Gezeigt ist dort lediglich die Wellenfront 27, die vom Ohrkanal 21 zurückreflektiert wird und den Aufnahmesensor 26 erreicht. Der Aufnahmesensor 26, z. B. ein CCD-Sensor, wird anstelle einer Fotoplatte bzw. eines Films 12 eingesetzt, so dass praktisch beliebig viele Aufnahmen ohne Film- bzw. Plattenwechsel aufgenommen werden können. Außerdem können die Daten von dem Aufnahmesensor direkt digital gewonnen und damit störungssicherer verarbeitet werden.

Die geometrische Anordnung und die optischen Eigenschaften der Komponenten des Aufnahmegeräts 20 bestimmen den maximalen Aufnahmewinkel ϕ. Da dieser Winkel ϕ in der Praxis nicht beliebig groß sein kann, ist es in der Regel notwendig, aus unterschiedlichen Positionen des Aufnahmegeräts 20 mehrere Einzelhologramme aufzunehmen und diese dann rechnerisch zu einem Gesamthologramm zusammenzusetzen. Hierzu ist es notwendig, dass das Aufnahmegerät im Ohrkanal unterschiedlich platziert und ausgerichtet werden kann. Folglich darf die Größe des Aufnahmegeräts 20 ein gewisses Höchstmaß nicht überschreiten, damit eine bestimmte Beweglichkeit in dem Ohrkanal gewährleistet bleibt.

Zur Bewegung des Aufnahmegeräts 20 ist an dessen Gehäuse 28 ein Führungselement 29 befestigt. Mit diesem Führungselement 29 ist es möglich, das Aufnahmegerät 20 entsprechend den in FIG 3 eingezeichneten Pfeilen zu kippen, zu rotieren und längs des Ohrkanals 21 zu bewegen. Das Führungselement kann beispielsweise ein flexibler Stab sein.

Das Hologramm-Aufnahmesystem kann grundsätzlich kleiner gebaut werden als ein üblicher 3D-Scanner, der auf dem Triangulationsprinzip beruht. Dieser 3D-Scanner erfordert nämlich einen Projektor und eine Kamera, die in einem spezifischen Winkel zueinander exakt ausgerichtet sein müssen. Das Hologramm-Aufnahemsystem hingegen benötigt lediglich einen Laser (Laserdiode) und einen dazu ausgerichteten halbtransparenten Spiegel, einen weiteren Umlenkspiegel sowie einen ebenfalls dazu ausgerichteten Aufnahmesensor. Das HologrammAufnahmesystem lässt sich so deutlich kleiner bauen als der 3D-Scanner. Obendrein benötigt der Hologrammsensor (CCD-Chip) keine Frontlinse, da er keine Abbildung eines Bildes, sondern Interferenzmuster auf seiner Oberfläche aufnimmt. Damit gibt es auch keine Probleme mit der Tiefenschärfe. Lediglich die Auflösung nimmt zum Rand hin geringfügig ab. Dies kann jedoch durch mehrere Aufnahmen aus verschiedenen Positionen ausgeglichen werden.

Die Messgenauigkeit des Hologramm-Aufnahmesystems hängt von der Lage des CCD-Chips 26 zu den Spiegeln 24, 25 und dem Laser 23 ab. Um die gewünschte Messgenauigkeit zu erreichen, ist eine entsprechende Kalibrierung notwendig. Dies kann grundsätzlich durch eine Einmalkalibrierung geschehen. Es kann aber auch in vorgegebenen Zeitabständen oder bei jeder Messung automatisch kalibriert werden. Hierzu wird ein Messreferenzobjekt bei der Hologrammaufnahme mit aufgenommen. Dieses Messreferenzobjekt besitzt eine bekannte Größe und Form, wie beispielsweise ein kleiner 3D-Stern. Anhand seines Hologramms lässt sich das gesamte übrige Hologramm kalibrieren und ein exaktes 3D-Bild im PC rekonstruieren. Hierzu wird in bekannter Weise das Hologramm in eine Punktewolke umgewandelt. Sofern es die PC-Rechenleistung zulässt, ist sogar eine vollautomatische Kalibrierung während der Messung anhand des Referenzobjekts möglich.

Anhand des im PC gewonnenen 3D-Bilds, das aufgrund der verschiedenen Aufnahmepositionen ohne wesentliche Abschattungen vorliegt, können sogar Haare im Gehörgang nachträglich erkannt und aus dem Bild bzw. der Datenvorlage für die Gehäuseherstellung entfernt werden, da das komplette 3D-Abbild des Ohrkanals ohne wesentliche Abschattungen vorliegen muss.

## Patentansprüche

1. Aufnahmevorrichtung zur Aufnahme der räumlichen Gestalt eines kompletten menschlichen Ohrkanals (21) mit
- einer Holographieeinheit(20), die eine Lichtquelle (23) aufweist und durch die ein Hologramm des Ohrkanals (21) erstellbar ist, indem die Holographieeinheit in den Ohrkanal (21) eingeführt wird,
**dadurch gekennzeichnet, dass**
- die Holographieeinheit (20) vollständig in den menschlichen Ohrkanal (21) einführbar ist,
- die Holographieeinheit eine halbtransparente Platte (24) zur Aufteilung des Lichtstrahls von der Lichtquelle (23) in einen Beleuchtungsstrahl und einen Referenzstrahl und einen Aufnahmesensor (26) zum Aufnehmen eines Objektstrahls, der durch Reflexion des Beleuchtungsstrahls an einem Teil des Ohrkanals entsteht, zusammen mit dem Referenzstrahl aufweist, und
- die Holographieeinheit ein Gehäuse (28)
aufweist, und mit einem an dem Gehäuse befestigten Führungselement (29) im Ohrkanal schwenkbar, rotierbar und längs des Ohrkanals verschiebbar ist.

2. Aufnahmevorrichtung nach Anspruch **1,** wobei durch die Holographieeinheit (20) mehrere Einzelhologramme aufnehmbar und zu dem Hologramm des Ohrkanals (21) zusammensetzbar sind.

3. Aufnahmevorrichtung nach einem der vorhergehenden Ansprüche, wobei mit der Holographieeinheit (20) ein digitales Hologramm des Ohrkanals (21) erstellbar ist.

4. Aufnahmevorrichtung nach einem der vorhergehenden Ansprüche, wobei die Holographieeinheit (20) einen CCD-Chip als Aufnahmesensor (26) aufweist.

5. Einrichtung zur Herstellung einer Gehäuseschale oder einer Otoplastik für eine Hörvorrichtung mit
- einer Aufnahmevorrichtung gemäß einem der vorhergehenden Ansprüche und
- einer Ausformungseinheit zum Formen der Gehäuseschale oder der Otoplastik aus dem durch die Aufnahmevorrichtung gewonnenen Hologramm des Ohrkanals (21).

6. Verfahren zum Aufnehmen der räumlichen Gestalt eines menschlichen Ohrkanals (21) durch
- Erstellen eines Hologramms des Ohrkanals (21), indem eine Holographieeinheit (20) in den Ohrkanal eingeführt wird, **dadurch gekennzeichnet, dass**
- die Holographieeinheit (20) vollständig in den Ohrkanal (21) eingeführt wird,
- ein Lichtstrahl einer Lichtquelle (23) der Holographieeinheit (20) in der Holographieeinheit (20) in einen Beleuchtungsstrahl und einen Referenzstrahl geteilt wird,
- ein Objektstrahl, der durch Reflexion des Beleuchtungsstrahls an einem Teil des Ohrkanals entsteht, zusammen mit dem Referenzstrahl im Ohrkanal durch einen Aufnahmesensor (26) der Holographieeinheit (20) als Hologramm aufgenommen wird, und
- die Holographieeinheit (20) für das Aufnehmen des Ohrkanals (21) mehrfach unterschiedlich im Ohrkanal (21) platziert und ausgerichtet wird.

7. Verfahren nach Anspruch **6,** wobei ein digitales Hologramm des Ohrkanals (21) erstellt wird.

8. Verfahren nach Anspruch **6** oder **7,** wobei mehrere Einzelhologramme aufgenommen und zu dem Hologramm des Ohrkanals (21) zusammengesetzt werden.

9. Verfahren nach einem der Ansprüche **6** bis **8,** wobei beim Aufnehmen des Ohrkanals (21) für das Hologramm ein Referenzobjekt mit aufgenommen wird.

10. Verfahren nach Anspruch **9,** wobei eine automatische Kalibrierung anhand des Referenzobjekts durchgeführt wird.

11. Verfahren zum Herstellen einer Gehäuseschale oder einer Otoplastik für.eine Hörvorrichtung durch
- Aufnehmen der räumlichen Gestalt eines menschlichen Ohrkanals (21) gemäß einem der Ansprüche **6** bis **10** und
- Formen der Gehäuseschale oder der Otoplastik aus dem gewonnenen Hologramm des Ohrkanals (21).

## Claims

1. Recording apparatus for recording the spatial structure of a complete human ear canal (21) having
- a holography unit (20), which comprises a light source (23) and by means of which a holograph of the ear canal (21) can be produced, by inserting the holography unit into the ear canal (21),
**characterised in that**
- the holography unit (20) can be completely inserted into the human ear canal (21),
- the holography unit comprises a semi-transparent disk (24) for dividing the light beam from the light source (23) into an illumination beam and a reference beam and comprising a recording sensor (26) for recording an object beam, which is produced by reflection of the illumination beam onto a part of the ear canal, together with the reference beam, and
- the holography unit comprises a housing (28), and can be pivoted, rotated and moved along the ear canal with a guide facility (29) fastened to the housing.

2. Recording apparatus according to claim 1, with it being possible to record a number of individual holograms by means of the holography unit (20) and to combine them to form a hologram of the ear canal (21).

3. Recording apparatus according to one of the preceding claims, with it being possible to produce a digital hologram of the ear canal (21) with the holography unit (20).

4. Recording apparatus according to one of the preceding claims, with the holography unit (20) comprising a CCD chip as a recording sensor (26).

5. Facility for producing a housing shell or an otoplastic for a hearing apparatus having
- a recording apparatus according to one of the preceding claims and
- a moulding unit for moulding the housing shell or the otoplastic from the hologram of the ear canal (21) obtained by means of the recording apparatus.

6. Method for recording the spatial structure of a human ear canal (21) by
- producing a hologram of the ear canal (21) by inserting a holography unit (21) into the ear canal,
**characterised in that**
- the holography unit (20) is completely inserted into the ear canal (21),
- a light beam of a light source (23) of the holography unit (20) in the holography unit (20) is divided into an illumination beam and a reference beam,
- an object beam, which is produced by reflection of the illumination beam onto a part of the ear canal, is recorded together with the reference beam as a hologram in the ear canal by means of a recording sensor (26) of the holography unit (20), and
- the holography unit (20) for recording the ear canal (21) is repeatedly positioned and aligned differently in the ear canal (21).

7. Method according to claim 6, with a digital hologram of the ear canal (21) being produced.

8. Method according to claim 6 or 7, with a number of individual holograms being recorded and being combined to form the hologram of the ear canal (21).

9. Method according to one of claims 6 to 8, with a reference object also being recorded during the recording of the ear canal (21) for the hologram.

10. Method according to claim 9, with an automatic calibration being carried out on the basis of the reference object.

11. Method for producing a housing shell or an otoplastic for a hearing apparatus by
- recording the spatial structure of a human ear canal (21) according to one of claims 6 to 10 and
- moulding the housing shell or the otoplastic from the obtained hologram of the ear canal (21).

## Revendications

1. Dispositif d'enregistrement pour l'enregistrement de la forme dans l'espace d'un canal ( 21 ) auriculaire humain complet, comprenant
- une unité d'holographie, qui comporte une source ( 23 ) lumineuse et par laquelle un hologramme du canal ( 21 ) auriculaire peut être établi en introduisant l'unité d'holographie dans le canal ( 21 ) auriculaire,
**caractérisé en ce que**
- l'unité ( 20 ) d'holographie peut être introduite complètement dans le canal ( 21 ) auriculaire humain,
- l'unité d'holographie comporte une lame ( 24 ) semi-transparente pour la répartition du faisceau lumineux de la source ( 23 ) lumineuse en un faisceau d'éclairage et en un faisceau de référence, et un capteur ( 26 ) de réception pour la réception d'un faisceau objet, qui se crée par réflexion du faisceau d'éclairage sur une partie du canal auriculaire, ensemble avec le faisceau de référence, et
- l'unité d'holographie comporte un boîtier ( 28 ) et peut basculer, tourner et se déplacer le long du canal auriculaire par un élément ( 29 ) de guidage fixé au boîtier.

2. Dispositif d'enregistrement suivant la revendication 1, dans lequel plusieurs hologrammes individuels peuvent être enregistrés et peuvent être composés en l'hologramme du canal ( 21 ) auriculaire par l'unité ( 20 ) d'holographie.

3. Dispositif d'enregistrement suivant l'une des revendications précédentes, dans lequel un hologramme numérique du canal ( 21 ) auriculaire peut être établi par l'unité ( 20 ) d'holographie.

4. Dispositif d'enregistrement suivant l'une des revendications précédentes, dans lequel l'unité ( 20 ) d'holographie comporte une puce CCD comme capteur ( 26 ) de réception.

5. Dispositif de production d'une coquille de boîtier ou d'un plastique auriculaire pour une prothèse auditive, comprenant
- un dispositif d'enregistrement suivant l'une des revendications précédentes et
- une unité de conformation pour conformer la coquille de boîtier ou le plastique auriculaire à partir de l'hologramme du conduit ( 21 ) auriculaire obtenu par le dispositif d'enregistrement.

6. Procédé d'enregistrement de la forme dans l'espace d'un canal ( 21 ) auriculaire humain, dans lequel
- on établit un hologramme du canal ( 21 ) auriculaire en introduisant l'unité ( 20 ) d'holographie dans le canal auriculaire,
**caractérisé en ce que**
- on introduit l'unité ( 20 ) d'holographie complètement dans le canal ( 21 ) auriculaire,
- on subdivise un faisceau lumineux d'une source ( 23 ) lumineuse de l'unité ( 20 ) d'holographie dans l'unité ( 20 ) d'holographie en un faisceau d'éclairage ou en un faisceau de référence,
- on enregistre, en tant qu'hologramme dans le canal auriculaire, par un capteur (26) de réception de l'unité ( 20 ) d'holographie, un faisceau objet qui se crée par réflexion du faisceau d'éclairage sur une partie du canal auriculaire ensemble avec le faisceau de référence, et
- on place et on dirige plusieurs fois de manière différente dans le canal ( 21 ) auriculaire l'unité ( 20 ) d'holographie pour l'enregistrement du canal ( 21 ) auriculaire.

7. Procédé suivant la revendication 6, dans lequel on établit un hologramme numérique du canal ( 21 ) auriculaire.

8. Procédé suivant la revendication 6 ou 7, dans lequel on enregistre plusieurs hologrammes individuels et on les compose en l'hologramme du canal ( 21 ) auriculaire.

9. Procédé suivant l'une des revendications 6 à 8, dans lequel, lors de l'enregistrement du canal ( 21 ) auriculaire, on enregistre pour l'hologramme un objet de référence.

10. Procédé suivant la revendication 9, dans lequel on effectue un étalonnage automatique au moyen de l'objet de référence.

11. Procédé de production d'une coquille de boîtier ou d'un plastique auriculaire pour une prothèse auditive, dans lequel
- on enregistre la forme dans l'espace d'un canal ( 21 ) auriculaire humain suivant l'une des revendications 6 à 10 et
- on forme la coquille de boîtier ou le plastique auriculaire à partir de l'hologramme du canal ( 21 ) auriculaire qui a été obtenu.
